# EUROPEAN PATENT SPECIFICATION

(11) **EP 3 209 356 B1**
(45) Date of publication and mention of the grant of the patent: **11.12.2019**
(21) Application number: 15784141.2
(22) Date of filing: 20.10.2015
(51) Int. Cl.: A61M 15/00, B65D 83/38, G01F 23/00

(54) **DISPENSING CONTAINER**
SPENDERBEHÄLTER
CONTENANT DE DISTRIBUTION

(30) Priority: 20.10.2014 GB 201418592
(43) Date of publication of application: 30.08.2017
(73) Proprietor: Trig1 Limited, Waterlooville P07 7SG (GB)
(72) Inventor: BACON, Raymond John, Waterlooville P07 7SG (GB); PEARCE, Stuart William, Blackburn Lancashire BB1 5RF (GB); NOBLE, Alex William, Blackburn Lancashire BB1 5RF (GB)
(74) Representative: Stratagem IPM Limited
(86) International application number: PCT/GB2015/053104
(87) International publication number: WO 2016/063020

(56) References cited:
- WO-A1-2011/095761
- CN-Y- 2 399 309
- US-A- 5 860 552

## Description

### FIELD

The present invention relates to a dispensing container particularly, but not exclusively, a dispensing container for storing and dispensing medicine.

### BACKGROUND

Dispensing containers come in many shapes and sizes. A particular problem associated with pressurised liquid dispensing containers, but not limited thereto, is the difficulty of determining the level of liquid present within the container. Such containers typically comprise a valve for dispensing liquid from the container and a vessel containing the liquid under pressure. The vessel is typically manufactured from impact extruded aluminium or rolled and welded sheet steel. Such materials are opaque making it impossible to see the quantity of liquid in the container. The quantity of liquid in the container also cannot easily be determined by handling, feel or sound of the container.

This problem is particularly evident in the medical field and particular in relation to inhalers used for respiratory conditions such as asthma. It is critical that a person suffering from a respiratory condition has access to inhalers to relieve symptoms such as being short of breath. Not being able to assess the liquid volume of a medicinal product in an inhaler container is problematic as the user of an inhaler would not be able to identify when they need to order a new inhaler or assess whether the inhaler has dispensed the number of doses that it contains. Thus it is not uncommon for person's suffering from respiratory conditions to either run out of their inhaler before they order a new one or order a new one and dispose of the old inhaler before it is empty thus wasting the contents.

WO2011/095761 provides solutions to the problem of not being able to determine the quantity of liquid in a pressurised liquid dispensing container.

The present invention seeks to provide an improved dispensing container for determining the quantity of liquid therein.

### SUMMARY

The present invention provides a dispensing container according to claim 1.

The provision of an additional formation or discrete element in or on the vessel to lift the vessel away from a surface when oriented in a lain-on-side attitude permits the container to roll under the influence of the centre of gravity of the container and/or any content therein even if the container encounters debris such as food crumbs, grit or hair, for example.

The additional formation or discreet element in or on the vessel for lifting the container away from a surface may take the form of at least one circumferential band, or tyre. Preferably, the at least one circumferential band, or tyre, comprises two circumferential bands or tyres. Extends around a portion of container

Use of an additional discreet element on the vessel, such as a circumferential band or tyre would enable such a band, or tyre, to be retrospectively installed to containers after manufacture.

The band, or tyre, may be positioned within a circumferential groove running around the vessel of the container.

The additional formation or discreet element in or on the vessel may alternatively take the form of an integral circumferential protuberance from the vessel or a 3D printed, or embossed logo.

The additional formation or discreet element in or on the vessel may further comprise an outwardly facing projection for preventing the container from settling in a certain orientation. The projection introduces instability into the container such that the centre of gravity of the content or the container will bias the container in the first orientation or second orientation respectively overcoming equilibrium in the case the centre of gravity of the content and centre of gravity of the container are equal.

### BRIEF DESCRIPTION OF THE DRAWINGS

Certain embodiments will now be described with reference to the following drawings:
Figure 1 shows a side view of a roll-stable container with an external groove positioned beneath the level of liquid therein;
Figure 2 shows a side view of the roll stable container of figure 1 with the groove positioned above the level of liquid therein;
Figure 3 shows a side view of the roll stable container of figures 1 and 2 with the groove effectively floating on the surface of the liquid therein;
Figure 4a shows a side view of a first roll stable container in accordance with the invention;
Figure 4b shows a detail view of part of the first roll stable container of figure 4a;
Figure 5a shows a side view of a second roll stable container in accordance with the invention;
Figure 5b shows a detail view of part of the second roll stable container of figure 5a;
Figure 6a shows a side view of a third roll stable container in accordance with the invention;
Figure 6b shows a detail view of part of the third roll stable container of 6a.

### DETAILED DESCRIPTION OF THE CERTAIN EMBODIMENTS

The certain embodiments will now be described, by way of example only, with reference to the brief description of the drawings.

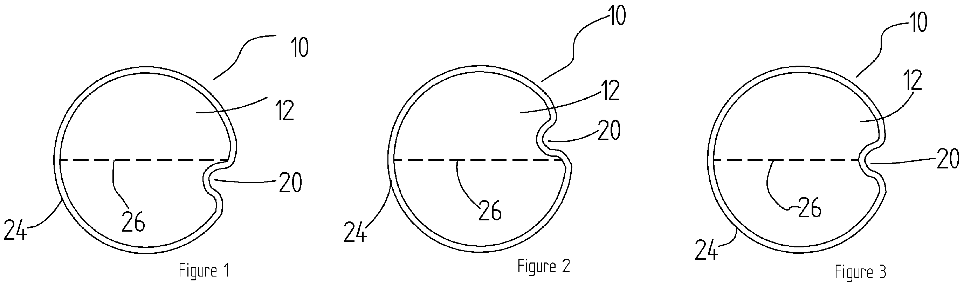

Referring to the figures, the dispenser 10 thereshown comprises a vessel 12 with a valve 14 crimped on at a neck 16 in the vessel. The valve has a dispensing stem 18. The vessel is circularly cylindrical and formed with a groove 20 within its circular cylindrical envelope, extending parallel with the length of the vessel giving it an inflected cylindrical sidewall. The arrangement is such that when the container is in an attitude of being lain on its sidewall 24, in which the groove 20 is, if the groove 20 is initially beneath the surface of the liquid 26 in the container, as shown in figure 1, the container 10 will roll, changing its orientation, until the groove 20 is at the surface of the liquid 26, as shown in figure 3, with the groove 20 in effect floating on the surface of the liquid 26. If the groove 20 is initially above the liquid 26, as shown in figure 2, since it biases the centre of gravity of the vessel 12 and the container 10 towards itself, being of constant wall thickness or having a bias weight in or on the vessel, the container 10 will roll again until the groove 20 is floating on the surface of the liquid 26, as shown in figure 3. Thus the extent of fullness of the container 10 can be determined by where the groove 20 comes to rest.

As shown in the figures, the groove 20 may extend only partially along the length of the vessel 12. The groove 20 may be oriented centrally in the longitudinal dimension of the vessel 12 or offset. In other embodiments the groove 20 may extend along the entire longitudinal dimension of the vessel from a base 22 to the neck 16.

When the liquid level is low, the groove 20 extending from end to end of the vessel 12 provides a particularly accurate indication of content, since liquid 26 cannot flow behind it.

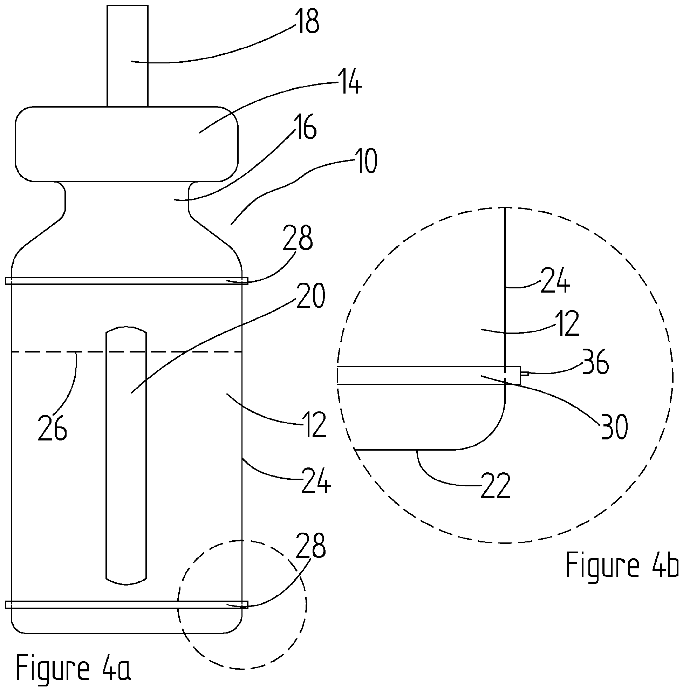

To assist the container 10 in being able to roll, a substantially circumferential formation 28 is provided at least at one end of the vessel 12 to lift the sidewall 24 of the vessel 12 away from a surface. Figures 4a and 4b show a roll-stable pressurised liquid dispenser 10 which has a tyre 30, or band, positioned around the vessel 12 at each end thereof. The tyre 30, or band, has a width of approximately 1mm and a depth of approximately 1mm. The tyre 30, or band, lifts the sidewall 24 of the vessel away from a surface by a distance of approximately 1mm. The tyre 30, or band, can be made from rubber, silicone, plastic, metal, or any other suitable material. The tyre 30, or band, has a smooth surface so as to reduce friction between the tyre 30, or band, and a surface. The formation 28 may extend around the entire circumference of the vessel 12 or only partially around the circumference of the vessel 12.

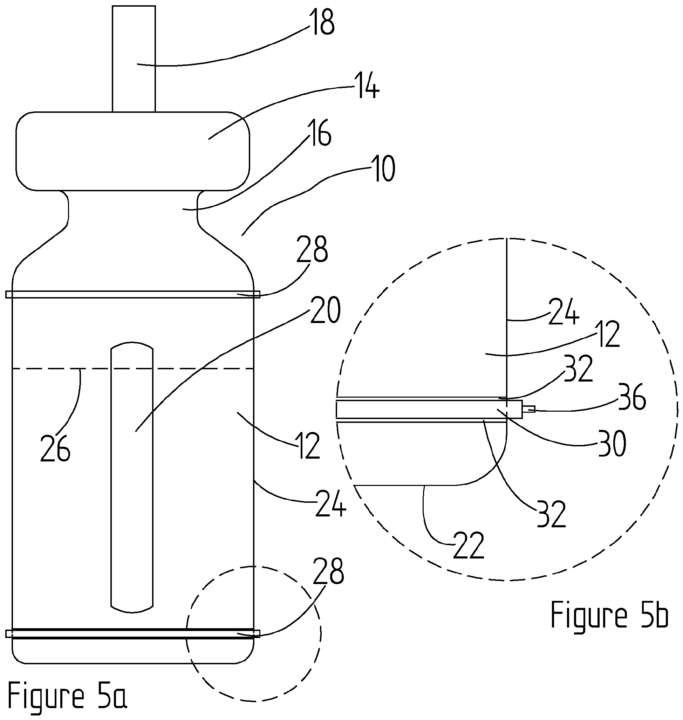

The tyre 30, or band, can be adhered to the vessel 12 or can utilise the elastic properties of the tyre 30, or band, to hold the tyre 30, or band, in the desired position. In some embodiments of the invention, the tyre 30, or band, can be positioned within a circumferential groove 32 in the sidewall 24 of the vessel 12, as shown in figures 5a and 5b.

In certain embodiments the formation 28 may be part of a label which is embossed or printed, for example to provide a raised area.

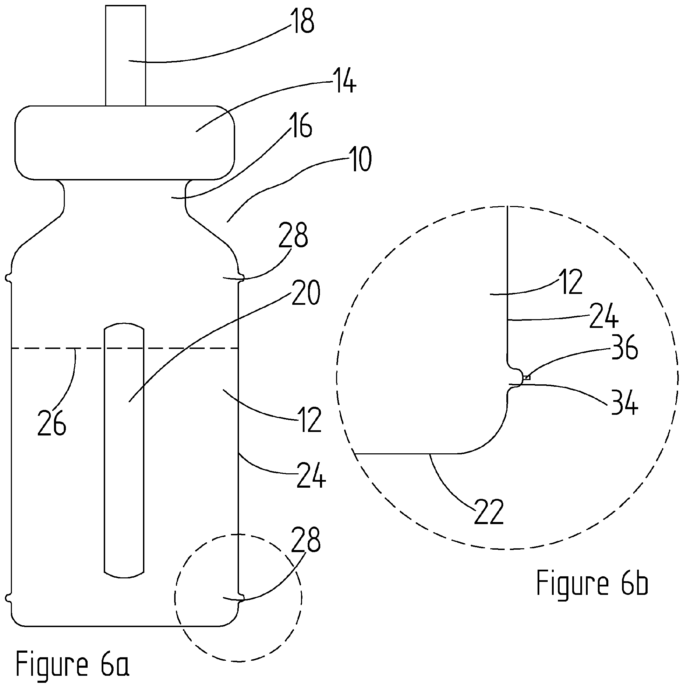

Figures 6a and 6b show a dispenser 10 which has an integral circumferential protuberance 34 formed in the sidewall 24 of the vessel 12. The protuberance defines a substantially uniformly curved surface which protrudes approximately 1mm from the sidewall 24 of the vessel 12.

As shown in figures 4b, 5b and 6b, the formation 28 is provided with a projection 36 which projects outwardly from the formation 28 to introduce an element of instability to the container. The projection 36 prevents the container 10 from settling at a dead centre orientation. In one embodiment the projection is a pin which has a length of between 1mm and 10mm and a width of between 0.5mm and 3mm. The pin is made from the same material as the circumferential protuberance, tyre or 3D printed label and is integral therewith. However, the skilled person will appreciate that in other embodiments the projection may be a separate component from the circumferential protuberance 34, tyre 30 or 3D printed label and maybe formed from a different material. The projection, in some embodiments, may also be a protuberance, spike or bump, for example.

## Claims

1. A dispensing container (10) comprising a vessel (12), the vessel having:
a groove (10) extending along the vessel which causes the centre of gravity of liquid contents of the vessel to be positioned differently, when oriented in a lain-on-side attitude, from its notional position, in the absence of the formation or element;
**characterised in that**:
the dispensing container further comprises a circumferential formation (28) arranged circumferentially around the vessel,
wherein, when the container is in the lain-on-side attitude and contains a first volume of content, the centre of gravity of the content biases the container to roll on the circumferential formation to a first orientation, and
when the container is in the lain-on-side attitude and has a second volume of content, the centre of gravity of the container biases the container to roll on the circumferential formation to a second orientation, different to the first,
**characterised in that** the circumferential formation is arranged to project from the vessel such that when the container is in the lain-on-side attitude, the circumferential formation lifts at least a portion of the container away from a surface to reduce the surface area of the container that is in contact with the surface.

2. A dispensing container (10) according to claim 1, wherein the circumferential formation(28) comprises at least one tyre (30), or band, arranged circumferentially around the vessel (12).

3. A dispensing container (10) according to claim 1, wherein the circumferential formation (28) comprises at least one integral circumferential protuberance (34).

4. A dispensing container (10) according to claim 2, wherein the tyre (30) also comprises a 3D printed, or embossed, label.

5. A dispensing container (10) according to claim 2 or 4, wherein the at least one tyre (30), or band, comprises two tyres, or bands.

6. A dispensing container (10) according to claim 5, wherein one tyre (30, or band, is positioned adjacent a base wall of the vessel (12) and the other tyre, or band, is positioned adjacent a neck portion of the vessel.

7. A dispensing container (10) according to claim 5 or claim 6, wherein each tyre (30), or band, is positioned within a circumferential groove (32) in a side wall of the vessel (12).

8. A dispensing container (10) according to claim 5 or claim 6, wherein each tyre (30), or band, is affixed to a sidewall of the vessel (12) by adhesive.

9. A dispensing container (10) according to any of claims 5 to 8, wherein each tyre (30), or band, is manufactured from rubber, silicone, plastic, metal, or any other suitable material.

10. A dispensing container (10) according to any of claims 5 to 9, wherein each tyre (30), or band, has a width of approximately 1mm and a depth of approximately 1mm.

11. A dispensing container (10) according to claim 3 wherein the at least one integral circumferential protuberance (34) comprises two integral circumferential protuberances.

12. A dispensing container (10) according to claim 11, wherein each integral circumferential protuberance (34) defines a substantially uniform curved surface.

13. A dispensing container (10) according to claims 11 or claim 12, wherein one integral circumferential protuberance (34) is positioned adjacent a base wall of the vessel and wherein the other integral circumferential protuberance is positioned adjacent a neck portion of the vessel.

14. A dispensing container (10) according to any preceding claim, wherein the circumferential formation (28) comprises an outwardly facing projection (36).

15. A dispensing container (10) according to claim 14, wherein the outwardly facing projection (36) comprises a pin.

## Patentansprüche

1. Spenderbehälter (10), der ein Gefäß (12) umfasst, wobei das Gefäß folgendes aufweist:
eine Rille (10), die sich entlang dem Gefäß erstreckt, was bewirkt, dass der Schwerpunkt des Flüssigkeitsinhalts des Gefäßes anders positioniert wird, wenn es aus seiner fiktiven Position in einer seitlichen Lage ausgerichtet ist, in Abwesenheit der Formation oder des Elements;
**dadurch gekennzeichnet, dass**:
der Spenderbehälter ferner eine umfängliche Formation (28) umfasst, die umfänglich um das Gefäß angeordnet ist;
wobei, wenn sich der Behälter in der seitlichen Lage befindet und ein erstes Inhaltsvolumen enthält, der Schwerpunkt des Inhalts den Behälter so vorbelastet, dass dieser auf der umfänglichen Formation an eine erste Ausrichtung rollt; und
wobei, wenn sich der Behälter in der seitlichen Lage befindet und ein zweites Inhaltsvolumen aufweiset, der Schwerpunkt des Behälters den Behälter so vorbelastet, dass dieser auf der umfänglichen Formation an eine zweite Ausrichtung rollt, die sich von der ersten Ausrichtung unterscheidet;
**dadurch gekennzeichnet, dass** die umfängliche Formation so angeordnet ist, dass sie von dem Gefäß vorsteht, so dass, wenn sich der Behälter in der seitlichen Lage befindet, die umfängliche Formation wenigstens einen Teil des Behälters von einer Oberfläche weg anhebt, um den Oberflächenbereich des Behälters zu reduzieren, der sich in Kontakt mit der Oberfläche befindet.

2. Spenderbehälter (10) nach Anspruch 1, wobei die umfängliche Formation (28) wenigstens einen Reifen (30) oder ein Band umfasst, das umfänglich um das Gefäß (12) angeordnet ist.

3. Spenderbehälter (10) nach Anspruch 1, wobei die umfängliche Formation (28) mindestens eine integrale umfängliche Protuberanz (34) umfasst.

4. Spenderbehälter (10) nach Anspruch 2, wobei das Rad (30) ferner ein mittels 3D-Druck erzeugtes oder geprägtes Etikett umfasst.

5. Spenderbehälter (10) nach Anspruch 2 oder 4, wobei der wenigstens eine Reifen (30) oder das Band zwei Reifen oder Bänder umfasst.

6. Spenderbehälter (10) nach Anspruch 5, wobei ein Reifen (30) oder Band angrenzend an eine Basiswand des Gefäßes (12) positioniert ist, und wobei der andre Reifen oder das andere Band angrenzend an einen Halsteil des Gefäßes positioniert ist.

7. Spenderbehälter (10) nach Anspruch 5 oder 6, wobei jeder Reifen (30) oder jedes Band in einer umfänglichen Rille (32) in einer Seitenwand des Gefäßes (12) positioniert ist.

8. Spenderbehälter (10) nach Anspruch 5 oder 6, wobei jeder Reifen (30) oder jedes Band durch Klebstoff an einer Seitenwand des Gefäßes (12) befestigt ist.

9. Spenderbehälter (10) nach einem der Ansprüche 5 bis 8, wobei jeder Reifen (30) oder jedes Band aus Kautschuk, Silikon, Kunststoff, Metall oder jedem anderen geeigneten Material hergestellt ist.

10. Spenderbehälter (10) nach einem der Ansprüche 5 bis 9, wobei jeder Reifen (30) oder jedes Band eine Breite von ungefähr 1 mm und eine Tiefe von etwa 1 mm aufweist.

11. Spenderbehälter (10) nach Anspruch 3, wobei die mindestens eine integrale umfängliche Protuberanz (34) zwei integrale umfängliche Protuberanzen umfasst.

12. Spenderbehälter (10) nach Anspruch 11, wobei jede integrale umfängliche Protuberanz (34) eine im Wesentlichen einheitlich gekrümmte Oberfläche definiert.

13. Spenderbehälter (10) nach Anspruch 11 oder 12, wobei eine integrale umfängliche Protuberanz (34) angrenzend an eine Basiswand des Gefäßes positioniert ist, und wobei die andere integrale umfängliche Protuberanz angrenzend an einen Halsteil des Gefäßes positioniert ist.

14. Spenderbehälter (10) nach einem der vorstehenden Ansprüche, wobei die umfängliche Formation (28) einen auswärts gerichteten Vorsprung (36) umfasst.

15. Spenderbehälter (10) nach Anspruch 14, wobei der auswärts gerichtete Vorsprung (36) einen Stift umfasst.

## Revendications

1. Contenant de distribution (10) comprenant un récipient (12), le récipient ayant :
une rainure (10) s'étendant le long du récipient, qui amène le centre de gravité du contenu liquide du récipient à être positionné différemment, lorsqu'il est orienté en position couchée sur le côté, de sa position théorique, en l'absence de la formation ou de l'élément ; **caractérisé en ce que** :
le contenant de distribution comprend en outre une formation circonférentielle (28) disposée circonférentiellement autour du récipient,
lorsque le contenant est dans la position couchée sur le côté et contient un premier volume de contenu, le centre de gravité du contenu sollicitant le contenant pour rouler sur la formation circonférentielle jusqu'à une première orientation, et
lorsque le contenant est dans la position couchée sur le côté et a un second volume de contenu, le centre de gravité du contenant sollicitant le contenant pour rouler sur la formation circonférentielle jusqu'à une seconde orientation, différente de la première,
**caractérisé en ce que** la formation circonférentielle est conçue pour faire saillie du récipient de sorte que, lorsque le contenant est dans la position couché sur le côté, la formation circonférentielle soulève au moins une partie du contenant à l'opposé d'une surface pour réduire l'aire de surface du contenant qui est en contact avec la surface.

2. Contenant de distribution (10) selon la revendication 1, la formation circonférentielle (28) comprenant au moins un pneumatique (30), ou bande, disposé circonférentiellement autour du récipient (12).

3. Contenant de distribution (10) selon la revendication 1, la formation circonférentielle (28) comprenant au moins une protubérance circonférentielle intégrale (34).

4. Contenant de distribution (10) selon la revendication 2, le pneumatique (30) comprenant également une étiquette imprimée en 3D ou estampée.

5. Contenant de distribution (10) selon la revendication 2 ou 4, l'au moins un pneumatique (30), ou bande, comprenant deux pneumatiques, ou bandes.

6. Contenant de distribution (10) selon la revendication 5, un pneumatique (30), ou bande, étant positionné près d'une paroi de base du récipient (12) et l'autre pneumatique, ou bande, étant positionné adjacent à une partie col du récipient.

7. Contenant de distribution (10) selon la revendication 5 ou 6, chaque pneu (30), ou bande, étant positionné dans une rainure circonférentielle (32) dans une paroi latérale du récipient (12).

8. Contenant de distribution (10) selon la revendication 5 ou 6, chaque pneu (30), ou bande, étant fixé à une paroi latérale du récipient (12) par adhésif.

9. Contenant de distribution (10) selon l'une quelconque des revendications 5 à 8, chaque pneu (30), ou bande, étant fabriqué en caoutchouc, silicone, plastique, métal ou tout autre matériau approprié.

10. Contenant de distribution (10) selon l'une quelconque des revendications 5 à 9, chaque pneumatique (30), ou bande, ayant une largeur d'environ 1 mm et une profondeur d'environ 1 mm.

11. Contenant de distribution (10) selon la revendication 3, l'au moins une protubérance circonférentielle intégrale (34) comprenant deux protubérances circonférentielles intégrales.

12. Contenant de distribution (10) selon la revendication 11, chaque protubérance circonférentielle intégrale (34) définissant une surface courbe sensiblement uniforme.

13. Contenant de distribution (10) selon la revendication 11 ou 12, une protubérance circonférentielle intégrale (34) étant positionnée adjacente à une paroi de base du récipient et l'autre protubérance circonférentielle intégrale étant positionnée adjacente à une partie col du récipient.

14. Contenant de distribution (10) selon l'une quelconque des revendications précédentes, la formation circonférentielle (28) comprenant une saillie faisant face à l'extérieur (36).

15. Contenant de distribution (10) selon la revendication 14, la saillie faisant face à l'extérieur (36) comprenant une broche.
